# EUROPEAN PATENT APPLICATION

(11) **EP 1 018 544 A1**
(43) Date of publication of application: **12.07.2000**
(21) Application number: 98928613.3
(22) Date of filing: 22.06.1998
(51) Int. Cl.: C12M 1/00, C12M 1/22, B65G 59/06, G01N 1/00

(54) **STOCKER FOR LABORATORY DISHES AND APPARATUS FOR TAKING OUT AND SUPPLYING LABORATORY DISHES**

(30) Priority: 23.06.1997 JP 16603797
(71) Applicant: Nittetsu Mining Co., Ltd., Tokyo 100-0005 (JP); SANKYO COMPANY LIMITED, Chuo-ku, Tokyo 103-0023 (JP)
(72) Inventor: INABA, Kazuhiro c/o Nittetsu Mining Co, Ltd., Nishitama-gun,Tokyo (JP); SHIRATORI, Mamoru-Sankyo Company, Limited, Tokyo 140-005 (JP); ICHIKAWA, Masato-Sankyo Company, Limited, Tokyo 140-0005 (JP)
(74) Representative: Grünecker, Kinkeldey, Stockmair & Schwanhäusser Anwaltssozietät
(86) International application number: JP9802772
(87) International publication number: WO9859033

(57) **Abstract**

The laboratory dish stocker (1) of the invention has: a pair of laboratory dish receiving pawls (7) which are placed in an opening edge of each of plural drop holes (6) opened in a base plate (2), so as to be swingable only in an upward direction, and which support a lower portion of a laboratory dish (S); plural support rods (9) which are placed in a periphery of each of the drop holes (6), and which support outer peripheries of laboratory dishes (S) stacked above the drop hole; and a handle (5) which is placed above the center of the base plate (2) via rods (3). The laboratory dish stocker (1) of the invention further has: an automatic laboratory dish extracting apparatus (20) which jumps the laboratory dish receiving pawls (7) of one of the drop holes (6) to extract a laboratory dish (S) from the lower side; and an automatic laboratory dish supplying apparatus (40) which jumps the laboratory dish receiving pawls (7) while pushing up a laboratory dish (S) into the drop hole (6), thereby accommodating the laboratory dish.

Therefore, a large number of laboratory dishes can be simultaneously accommodated, and a laboratory dish can be easily extracted without breaking the stack of accommodated laboratory dishes.

## Description

### Technical Field

The present invention relates to a laboratory dish stocker which can hold a large number of laboratory dishes in a stacked manner and allows the laboratory dishes to be transported and/or tested, an automatic laboratory dish extracting apparatus which can sequentially extract the laboratory dishes one by one from the laboratory dish stocker into a testing apparatus, and an automatic supplying apparatus for sequentially extracting laboratory dishes one by one from a testing apparatus and causing the laboratory dishes to be again held in the laboratory dish stocker in a stacked manner.

### Background Art

In the case of an automatic test on a microorganism and using laboratory dishes or of automatic production and processing of a medium such as an agar medium, conventionally, a method is usually employed in which a rack accommodating a large number of laboratory dishes in a row and is placed in a supply position, the laboratory dishes are supplied to a test or process position while being extracted one by one from the rack, the laboratory dishes that have been tested or processed are again accommodated into the rack in a stacked manner, and the laboratory dishes are then transported to the next test or process position.

In the supply and transport method using a rack, however, each rack has a limited maximum number of accommodated laboratory dishes, and hence the number of accommodated laboratory dishes is not large. This problem may be solved by making a rack taller. However, such a rack is unstable during transportation.

Since a rack has a box-like shape, it is difficult to externally check the specimen number of each of stacked laboratory dishes. When a laboratory dish placed in an intermediate position is to be extracted from a rack, furthermore, the laboratory dish cannot be extracted unless all laboratory dishes placed above the laboratory dish are extracted from the rack to break the stacked state. Hence, there are disadvantages that it is cumbersome to perform a random sampling inspection or extract a laboratory dish of a specific specimen ID, and that laboratory dishes are often stacked in an incorrect order in the case where the laboratory dish is extracted or reaccommodated.

The invention has been conducted in order to solve the above-discussed problems. It is an object of the invention to provide a laboratory dish stocker which can simultaneously accommodate a large number of laboratory dishes in a stacked manner, and from which a laboratory dish in the middle of the stack can be easily extracted without breaking the stack of laboratory dishes.

It is another object of the invention to provide an automatic laboratory dish inserting/extracting apparatus which uses the laboratory dish stocker, and which is suitable for sequentially extracting laboratory dishes from the laboratory dish stocker to feed the laboratory dishes to an automatic machine, and reaccommodating the laboratory dishes from the automatic machine into the laboratory dish stocker.

### Means for Solving the Problems

In order to attain the objects, the laboratory dish stocker of the invention is characterized in that the laboratory dish stocker comprises: a base plate in which plural laboratory dish drop holes are opened; at least one pair of laboratory dish receiving pawls which are placed in an opening edge of each of the drop holes so as to be swingable only in an upward direction, and which support a lower portion of one of laboratory dishes stacked above the drop hole; plural support rods which stand from a face of the base plate and in a periphery of each of the drop holes, and which support outer peripheries of laboratory dishes stacked above the drop hole; and a handle which is placed above a center of the base plate via a rod.

According to this configuration, a large number of laboratory dishes can be carried and extracted in a sure, safe, and easy manner. Each laboratory dish can be externally observed through spaces between the support rods. Therefore, sample numbers or the like can be easily checked.

In the configuration, the drop holes may be configured so as to be communicatingly opened in an outer periphery of the base plate via respective cutaways.

In this case, when a laboratory dish is to be extracted into an automatic extracting apparatus which will be described later, the laboratory dish can be easily detected, and, when the laboratory dish at the lowest position is to be manually extracted, the laboratory dish can be easily extracted by inserting a finger into this portion.

A configuration may be employed in which an upper end of each of the support rods is formed as a free end, and the support rods are bendable so as to enlarge a rod space in the vicinity of middles of the support rods to a degree which is substantially equal to a diameter of the laboratory dish. According to this configuration, when a laboratory dish in an intermediate position of the stack is to be manually extracted, the rod space is widened, so that the laboratory dish can be extracted from an intermediate position of the rods. As a result, it is not required to move laboratory dishes which are stacked above the target laboratory dish, to the tip ends of the rods.

The automatic extracting apparatus according to the invention is characterized in that the apparatus comprises: a stationary table which is placed above a transportation path for a laboratory dish and in which a receiving hole is opened at one position, the receiving hole being communicable with drop holes of a laboratory dish stocker; an indexing table which is placed on the stationary table in a stepwise rotatable manner, on which the laboratory dish stocker is placed, and in which intermediate holes are opened, the intermediate holes being coincidable with the drop holes of the laboratory dish stocker and the receiving hole of the stationary table; and abutting pins which are projected from a periphery of each of the intermediate holes of the indexing table, and which are to abut against laboratory dish receiving pawls of the laboratory dish stocker to outward jump the laboratory dish receiving pawls, thereby causing a laboratory dish to drop.

According to this configuration, it is possible to sequentially drop a large number of laboratory dishes which are held on the laboratory dish stocker, one by one toward the transportation path.

Preferably, a center positioning pin and a position detecting pin are projected at a center position and an appropriate peripheral position of the indexing table, the center positioning pin and the position detecting pin being to be respectively passed through a center hole and an indexing hole which are respectively opened at a center position and an appropriate peripheral position of a base plate of the laboratory dish stocker.

According to this configuration, the work of positioning the laboratory dish stocker with respect to the indexing table can be simplified.

A configuration may be employed in which the intermediate holes of the indexing table are communicatingly opened in an outer periphery via respective cutaways, and a non-contact sensor is disposed in the vicinity of the receiving hole of the stationary table, the non-contact sensor detecting presence or absence of a laboratory dish via cutaways which are opened in the indexing table and an outer periphery of a base plate of the laboratory dish stocker.

According to this configuration, presence or absence of a laboratory dish which is to be extracted toward the transportation path is detected, and, in accordance with a result of the detection, indexing rotation of the indexing table, an alarm output in the case of empty, and the like are performed.

The automatic supplying apparatus of the invention comprises: a stationary table which is placed in an end position of transportation for a laboratory dish and in which an extracting hole is opened at one position, the extracting hole being communicable with drop holes of a laboratory dish stocker; an indexing table which is placed on the stationary table in a stepwise rotatable manner, and in which intermediate holes are opened, the intermediate holes being coincidable with the drop holes of the laboratory dish stocker and a receiving hole of the table; and pushing-up means for protruding below the transportation end position in an extractable and retractable manner, to push up a laboratory dish supplied to the position, at least to a position above laboratory dish receiving pawls.

According to this configuration, in a state of pushing toward the laboratory dish stocker, the laboratory dish receiving pawls are jumped, and then receives the lower face of a laboratory dish. Therefore, laboratory dishes can be sequentially supplied in a stacked manner from a bottom portion of the laboratory dish stocker.

Preferably, a center positioning pin and a position detecting pin are projected at a center position and an appropriate peripheral position of the indexing table, the center positioning pin and the position detecting pin being to be respectively passed through a center hole and an indexing hole which are respectively opened at a center position and an appropriate peripheral position of a base plate of the laboratory dish stocker.

Also in this configuration, the same effects as those of the supplying apparatus can be attained.

### Brief Description of Drawings

Fig. 1 is an exploded perspective view of a laboratory dish stocker according to the invention.
Fig. 2 is a perspective view showing an assembled state of the laboratory dish stocker.
Fig. 3 is a perspective view showing a state where laboratory dishes are stacked in the laboratory dish stocker.
Fig. 4 is an exploded perspective view of an automatic laboratory dish inserting apparatus.
Fig. 5 is a perspective view showing relationships between the inserting apparatus and a laboratory dish stocker.
Figs. 6(a) to (c) are partial section views showing a procedure of inserting a laboratory dish into the inserting apparatus.
Figs. 7(a) to (c) are partial section views showing a procedure of extracting a laboratory dish by an extracting apparatus with respect to the laboratory dish stocker.

### Mode for Carrying Out the Invention

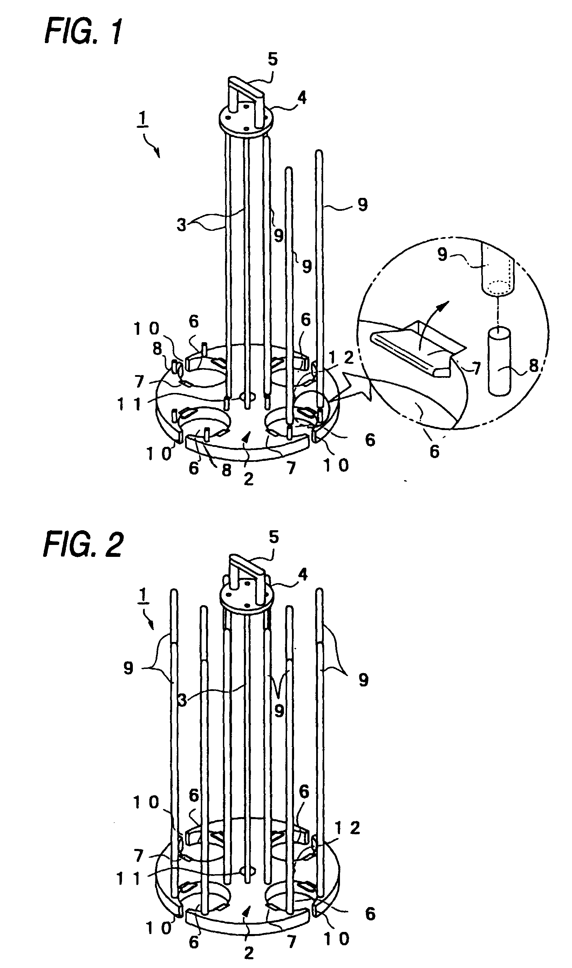

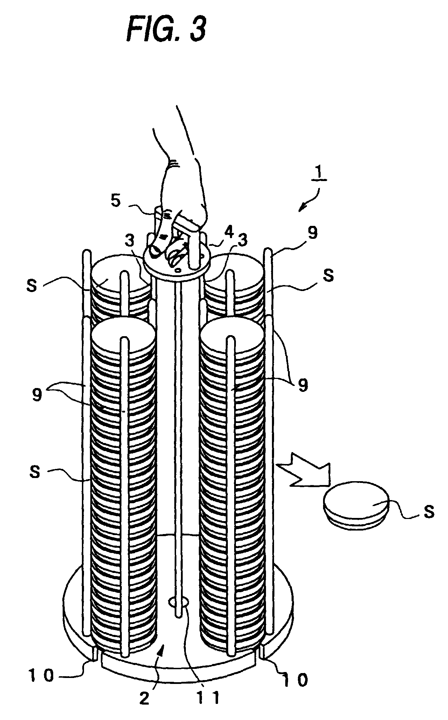

Hereinafter, preferred embodiments of the invention will be described in detail with reference to the accompanying drawings. Figs. 1 and 2 show a laboratory dish stocker 1 of the invention, and Fig. 3 shows a state where a large number of laboratory dishes S are stacked in the laboratory dish stocker 1.

The laboratory dish stocker 1 shown in the figures comprises: a base plate 2 which is formed of a stainless steel plate or the like and into a disk-like shape; an upper disk 4 which is coupled to an upper center position of the base plate 2 via plural rods 3, and which has a smaller diameter; and a handle 5 which is integrally projected from the upper face of the upper disk 4, and which is used for carrying the stocker.

Four circular drop holes 6 through which a laboratory dish S can freely pass are opened in the base plate 2. As enlargedly shown in a part of Fig. 1, a pair of laboratory dish receiving members 7 for supporting the lower face of a laboratory dish S and having a hook-like section shape are pivotally supported in a slightly upward attitude, in diagonal positions of the inner periphery of each of the drop holes 6. The tip ends of the members are projected into the inner periphery of the drop hole 6.

The receiving members 7 are swingable only toward an upper position with respect to their projection positions. When a laboratory dish S is supplied from the lower side, the members are opened so as to allow the laboratory dish to pass thereover, and then returned to the illustrated positions by elasticity of springs which are not shown. By contrast, even when a laboratory dish is tried to pass over the members from the upper side, the members maintain the state where they are projected into the inner periphery of the drop hole 6, thereby supporting the lower face of the laboratory dish S.

The reason why the receiving members 7 are placed in a slightly upward attitude is as follows. In the case where the receiving members 7 are opened and a laboratory dish S is to be received to a stacker position, when the receiving members are horizontally placed, edges of the receiving members 7 are in frictional contact with an outer peripheral portion of the laboratory dish S, and hence the outer periphery of the laboratory dish S is damaged. By contrast, when the receiving members are placed with being slightly upward directed, the frictional contact is reduced, and hence such a damage is prevented from occurring. When the surface of each receiving member 7 is lined with a lubricant resin such as Teflon, the effect of preventing a damage is further enhanced.

Four pins 8 are projected from an outer peripheral portion of each of the drop holes 6. Support rods 9 for supporting outer peripheral portions of laboratory dishes S are respectively fittingly fixed to the pins 8 so as to stand on the base plate 2.

In the figures, only three support rods 9 which are required at a minimum are shown. Although the number is not particularly restricted, the three support rods can sufficiently stack support laboratory dishes S. When the support rods 9 are made of a material such as stainless steel or aluminum and configured so that their bases are fixed in a loose condition of a certain degree, the spaces among the rods can be provided with degree of freedom. Alternatively, the rods may be made of an elastic material.

A cutaway 10 which communicates with the outer periphery of the base plate 2 is formed in each of the drop holes 6. The cutaway 10 functions to allow a finger to be inserted thereinto so as to facilitate extraction of the laboratory dish S located in the lowest position, and also to enable detection of a laboratory dish in an automatic laboratory dish inserting apparatus which will be described later.

The reference numeral 11 denotes a center positioning hole which is opened at the center of the base plate 2 so as to vertically pass therethrough, and 12 denotes an indexing hole which is opened at an appropriate position of the base plate 2. When the stocker is to be set to an inserting apparatus or an extracting apparatus, these holes are used for positioning.

In the above configuration, when the laboratory dish stocker 1 is to be carried, the stocker can be carried by hand with grasping the handle 5 as shown in Fig. 3. When, among many stacked laboratory dishes S, a laboratory dish S placed in the vicinity of an intermediate position is to be extracted, the laboratory dish can be pulled out through a gap between the support rods 9 while opening the support rods 9, because the bases of the support rods 9 are provided with degree of freedom as described above.

Such a use mode may be employed in the case of a sampling inspection, or an inspection in which a laboratory dish S provided with a specific ID number is checked. After the laboratory dish is pulled out, the support rods 9 elastically returns to their original positions. During a usual use state, therefore, the laboratory dishes S are restrained at their outer peripheries by the support rods 9, and, even when the stocker is slightly tilted, the laboratory dishes are kept in the stacked state.

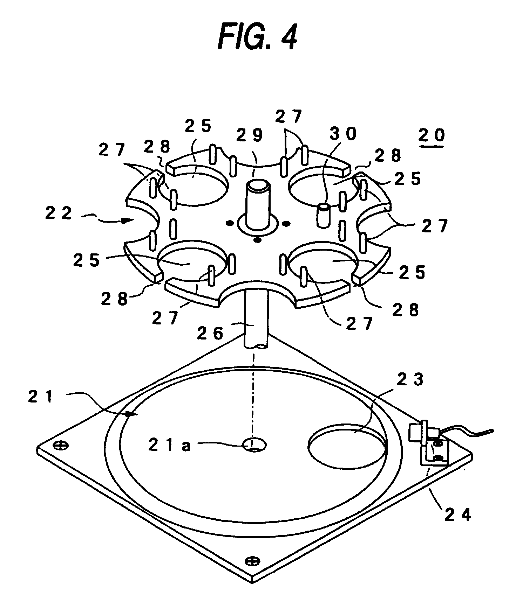

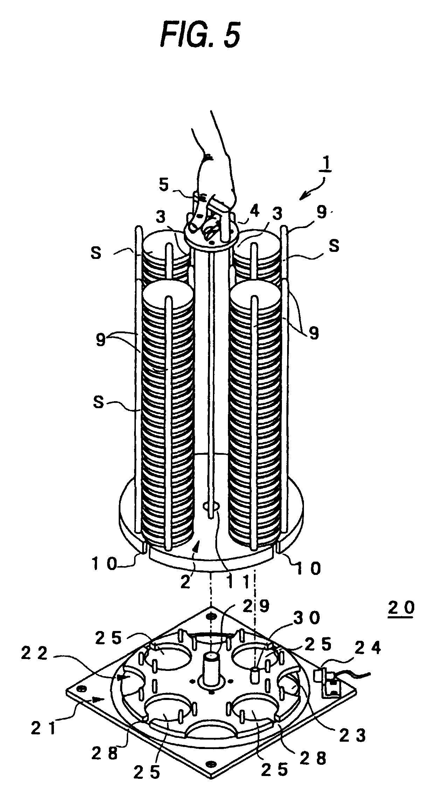

Figs. 4 and 5 show the above-mentioned automatic laboratory dish extracting apparatus 20 which pulls out a laboratory dish S from the laboratory dish stocker 1 and then supplies the laboratory dish to an inspecting and producing apparatus.

The automatic extracting apparatus 20 comprises: a stationary table 21 which has a rectangular plate-like shape, and which is placed above a laboratory dish transportation path (described later) so as to cover the upper portion thereof; and an indexing table 22 which is rotatably placed on the upper face of the stationary table 21.

At one position of the stationary table 21, a receiving hole 23 is disposed which is coincidable with the drop holes 6 of the laboratory dish stocker 1, and which is opened directly above the laboratory dish transportation path. A photoelectric sensor 24 which detects presence or absence of a laboratory dish S is disposed on the stationary table 21 and in the vicinity of the receiving hole 23.

In the indexing table 22, four intermediate holes 25 the diameter of which is substantially equal to that of the base plate 2 of the laboratory dish stocker 1, and which are equal in number to the drop holes 6 opened in the base plate 2 are opened. The indexing table is pivotally supported via an indexing driving shaft 26 in a center shaft hole 21a of the stationary table 21 so as to be rotatable.

The lower side of the driving shaft 26 is linked to an indexing driving motor which is not shown.

Each of the intermediate holes 25 is set to have a size which is larger than the outer diameters of the laboratory dishes S and smaller than the inner diameters of the drop holes 6. Two pair of abutting pins 27 which are to abut against the receiving members 7 to upward swing them are projected from an outer peripheral portion of the intermediate hole 25.

In each of the intermediate holes 25, furthermore, a cutaway 28 which is opened in the outer peripheral portion of the indexing table 22 is formed, so as to enable the photoelectric sensor 24 to detect presence or absence of the laboratory dishes S.

Furthermore, a center positioning pin 29 which passes through the center positioning hole 11 of the laboratory dish stocker 1 is upward projected from the center of the indexing table 22, and an indexing pin 30 for the indexing holes 12 is upward projected from an appropriate position of the table.

When the laboratory dish stocker 1 is to be disposed on the indexing table 22 as shown in Fig. 5, therefore, the center positioning hole 11 and one of the indexing holes 12 of the base plate 2 are positioned so as to respectively coincide with the pins 29 and 30, and the stocker can be then disposed on the indexing table 22 while the drop holes 6 accurately coincide with the intermediate holes 11, respectively.

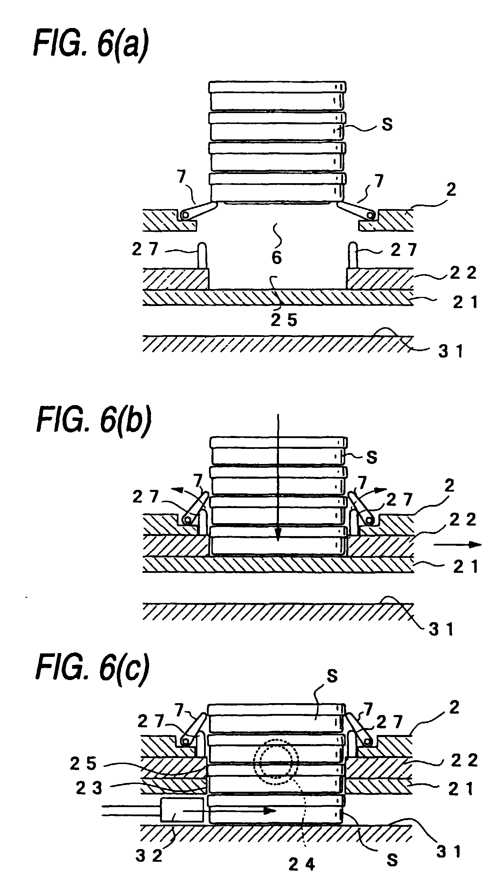

Fig. 6 shows a procedure of receiving the laboratory dish S into the extracting apparatus 20 during the disposition. As shown in (a), when the base plate 2 of the laboratory dish stocker 1 is upward separated from the indexing table 22, the laboratory dish S at the lowest position of the stacker is supported by the receiving members 7, and the outer periphery is restrained by the support rods 9. As a result, the whole of the laboratory dishes S is stacked in an aligned manner.

As shown in (b), when the base plate 2 is moved from the above state so as to sit on the indexing table 22, the abutting pins 27 abut against the receiving members 7 to outward swing them. As a result, the receiving members 7 are disengaged from the laboratory dish S, so that the laboratory dishes S drop in a lump into the intermediate hole 25 and the lowest laboratory dish S sits on the surface of the stationary table 21.

Under this state, the indexing table 22 is rotated. When the intermediate hole coincides with the receiving hole 23 opened in the stationary table 21, the rotation is stopped.

Under this coincidence state, as shown in (c), the laboratory dishes S drop in a lump through the receiving hole 23 onto the transportation face of the transportation path 31 in the same manner as the above-mentioned case. The laboratory dishes are sequentially transported one by one toward a process position by reciprocal operation of a pushing apparatus 32 which is disposed at the start end of the transportation path 31. In the figure, broken lines indicate the photoelectric sensor 24 which always detects presence or absence of a supply of the laboratory dish S.

Alternatively, means for, during a time when the lowest laboratory dish S is pushed out by the pushing apparatus 32, holding the next laboratory dish S at the upper position may be disposed in the transportation path 31. According to this configuration, the pushed-out laboratory dishes S are sequentially fed without being adversely affected by the gravity of the whole or friction.

As a result of the sequential feeding operation, the laboratory dishes S are sequentially sent with starting from the lower side, to an automatic machine via the inserting apparatus 20. When all the laboratory dishes S in this position are extracted away, the photoelectric sensor 24 detects this empty state.

In accordance with a result of this detection, the pushing apparatus 32 retracts to the waiting position, and the indexing table 22 then makes 1/4 rotation. As a result, the same sequential feeding operation as that described above is enabled.

When this work is repeated four times, a control apparatus or the like outputs an alarm indicating that the laboratory dish stocker 1 is empty, and stops the work.

When the laboratory dish stocker 1 which is made empty is detached from the indexing table 22 at this timing, therefore, it is possible to supply the next laboratory dish stocker 1 to the supply operation.

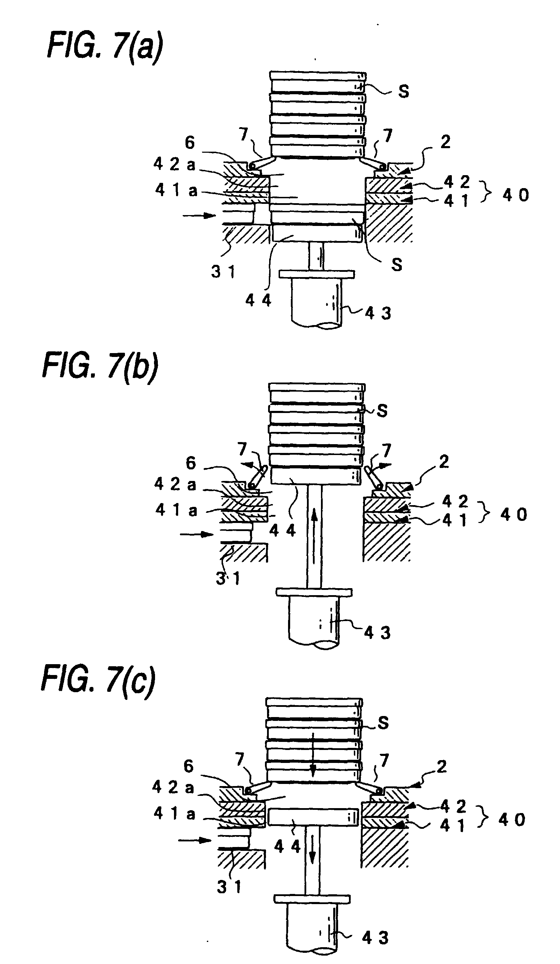

Next, an automatic laboratory dish supplying apparatus 40 in the transportation end position will be described with reference to Figs. 7(a) to (c). The supplying apparatus 40 comprises: a stationary table 41 which is placed in the transportation end position; and an indexing table 42 which is rotatably placed on the stationary table 41.

The stationary table 41 and the indexing table 42 are configured in the same manner as those of the extracting apparatus 20 shown in Figs. 4 and 5 except that the abutting pins 27 are not projectingly disposed on the indexing table 42. Therefore, their detailed description is omitted. A hole 41a which is opened in the stationary table 41 is referred to as an extracting hole, but holes 41a which are opened in the indexing table 42 are referred to as described above, or intermediate holes.

A liftable pushing apparatus 43 which waits in a state where the upper end is positioned so as to be flush with the transportation face is disposed below the end position of the transportation path 31 in the apparatus. When a laboratory dish S is supplied to the position of an upper seat plate 44 of the pushing apparatus 43 as shown in (a), the pushing apparatus 43 is raised so as to supply the laboratory dish S toward the laboratory dish stocker 1.

As shown in (b), the maximum raised position is set to a position where the lower face of the laboratory dish S is slightly higher than the upper edges of the receiving members 7 which are outward swung. The receiving members 7 are outward swung by the pushed-up laboratory dish S itself.

When the pushing apparatus 43 is lowered under this state, the whole is pushed down by the gravity of the laboratory dishes S, and the receiving members 7 are again inward swung so as to support the lower face of the pushed down laboratory dish S. In this way, laboratory dishes S are sequentially held in a stacked state by the laboratory dish stocker 1.

The above-described operation may be performed in the following manner. The number of raising and lowering operations of the pushing apparatus 43 is counted. When the counted number reaches a predetermined number, the work is stopped. Alternatively, according to a combination of the above and a photoelectric sensor, the stacking operation, the separated rotation, and the like may be performed. When a specified number of laboratory dishes S are accommodated in all the four stack positions of the laboratory dish stocker 1, the work is ended.

### Industrial Applicability

As apparent from the above description, the laboratory dish stocker and the automatic laboratory dish extracting/supplying apparatus of the invention have the following advantages.
(1) Since plural columns of laboratory dishes can be held in a stacked state, a large number of laboratory dishes can be carried and extracted. Since each laboratory dish can be externally observed, sample numbers or the like can be easily checked.
(2) When a laboratory dish is to be extracted into an automatic extracting apparatus, the laboratory dish can be easily detected, and, when the laboratory dish at the lowest position is to be manually extracted, the laboratory dish can be easily extracted by inserting a finger into this portion.
(3) When a laboratory dish in an intermediate position of the stack is to be manually extracted, extraction of the laboratory dish is enabled by elastically deforming the rods.
(4) In the automatic extracting apparatus of the invention, it is possible to sequentially drop a large number of laboratory dishes which are held on the laboratory dish stocker, one by one toward the transportation path.
(5) In the automatic extracting apparatus and the supplying apparatus, the work of positioning the laboratory dish stocker with respect to the indexing table can be simplified.
(6) The presence or absence of a laboratory dish which is to be extracted toward the transportation path is detected, and, in accordance with a result of the detection, indexing rotation of the indexing table, an alarm output in the case of empty, and the like are performed.
(7) In the automatic supplying apparatus of the invention, in a state of pushing toward the laboratory dish stocker, the laboratory dish receiving pawls are jumped, and then receives the lower face of a laboratory dish. Therefore, laboratory dishes can be sequentially supplied in a stacked manner from a bottom portion of the laboratory dish stocker.

## Claims

1. A laboratory dish stocker (1) characterized in that said stocker comprises:
a base plate (2) in which plural laboratory dish drop holes (6) are opened;
at least one pair of laboratory dish receiving pawls (7) which are placed in an opening edge of each of said drop holes (6) so as to be swingable only in an upward direction, and which support a lower portion of one of laboratory dishes (S) stacked above said drop hole (6);
plural support rods (9) which stand from a face of said base plate and in a periphery of each of said drop holes (6), and which support outer peripheries of laboratory dishes (S) stacked above said drop hole (6); and
a handle (5) which is placed above a center of said base plate (2) via a rod (3).

2. The laboratory dish stocker according to claim 1, wherein said drop holes (6) are communicatingly opened in an outer periphery of said base plate (2) via respective cutaways (10).

3. The laboratory dish stocker according to claim 1, wherein an upper end of each of said support rods (9) is formed as a free end, and said support rods (9) are flexible so as to enlarge a rod space in the vicinity of middles of said support rods to a degree substantially equal to a diameter of the laboratory dish (S).

4. An automatic laboratory dish extracting apparatus (20) on which the laboratory dish stocker according to claim 1 is to be placed, and which is characterized in that said apparatus comprises:
a stationary table (21) which is placed above a transportation path for a laboratory dish (S) and in which a receiving hole (23) is opened at one position, said receiving hole being communicable with said drop holes (6) of said laboratory dish stocker (1);
an indexing table (22) which is placed on said stationary table (21) in a stepwise rotatable manner, on which said laboratory dish stocker (1) is placed, and in which intermediate holes (25) are opened, said intermediate holes being coincidable with said drop holes (6) of said laboratory dish stocker (1) and said receiving hole (23) of said stationary table (21); and
abutting pins (27) which are projected from a periphery of each of said intermediate holes (25) of said indexing table (22), and which are to abut against said laboratory dish receiving pawls (7) of said laboratory dish stocker (1) to outward jump said laboratory dish receiving pawls (7), thereby causing a laboratory dish (S) to drop.

5. The automatic extracting apparatus according to claim 4, wherein said intermediate holes (25) of said indexing table (22) are communicatingly opened in an outer periphery via respective cutaways, and a non-contact sensor (24) is disposed in the vicinity of said receiving hole (23) of said stationary table (21), said non-contact sensor detecting presence or absence of a laboratory dish (S) via cutaways (10, 28) which are opened in said indexing table (22) and an outer periphery of said base plate (2) of said laboratory dish stocker (1).

6. An automatic laboratory dish supplying apparatus (40) on which the laboratory dish stocker according to claim 1 is to be placed, and which is characterized in that said apparatus comprises:
a stationary table (41) which is placed in an end position of transportation for a laboratory dish (S) and in which an extracting hole is opened at one position, said extracting hole being communicable with said drop holes (6) of said laboratory dish stocker (1); an indexing table (42) which is placed on said stationary table (41) in a stepwise rotatable manner, and in which intermediate holes (42a) are opened, said intermediate holes being coincidable with said drop holes (6) of said laboratory dish stocker (1) and said extracting hole (41a) of said stationary table (41); and pushing-up means (43) for protruding below the transportation end position in an extractable and retractable manner, to jump a laboratory dish supplied to the position, at least to a position above said laboratory dish receiving pawls.
